# EUROPEAN PATENT APPLICATION

(11) **EP 1 306 446 A1**
(43) Date of publication of application: **02.05.2003**
(21) Application number: 00949954.2
(22) Date of filing: 02.08.2000
(51) Int. Cl.: C12Q 1/68, C12N 15/10, G01N 33/50

(54) **KITS FOR EXTRACTING NUCLEIC ACID AND METHOD OF EXTRACTING NUCLEIC ACID BY USING THE KITS**

(71) Applicant: ORIENTAL YEAST CO., LTD., Tokyo 174-8505 (JP); JAPAN as represented by DIRECTOR GENERAL OF NATIONAL INSTITUTE OF INFECTIOUS DISEASES, Tokyo 162-8640 (JP)
(72) Inventor: YOSHIHARA, Namiko, Nerima-ku, Tokyo 176-0021 (JP); SUZUKI, Hisako, Omiya-shi, Saitama 330-0038 (JP); NAKAMURA, Taichi, Kitaadachi-gun, Saitama 362-0806 (JP); MANABE, Sachiko, Taito-ku, Tokyo 110-0014 (JP)
(74) Representative: HOFFMANN - EITLE
(86) International application number: JP0005170
(87) International publication number: WO02012559

(57) **Abstract**

The present invention provides a nucleic acid isolation kit comprising a reducing agent, a coprecipitant and a protein denaturant characterized in that it contains no protease, and a method for isolating a nucleic acid using said kit.

## Description

### TECHNICAL FIELD

The present invention relates to nucleic acid isolation kits and nucleic acid isolation methods using said kits.

### BACKGROUND ART

Detection of target nucleic acids such as specific DNA or RNA in biological samples such as blood and saliva is very important not only in the field of research but also in the clinical field.

For example, the rapid increase in the number of patients with human immunodeficiency virus (HIV) infection or AIDS in recent years is a matter of concern not only in Japan but also worldwide. A recent epidemiological tendency of HIV infection is a marked increase in the proportions of vertical infection or infection at younger ages. In the clinical field, there is an earnest demand for the development of an accurate and rapid genetic diagnosis. In Japan, the main infection route of HIV infection or AIDS especially in hemophiliacs is thought to be transfusion of blood including blood products. However, little detail has been elucidated for the infection route from blood products (Lin Qi Zhang, Peter Simmonds, Christopher A. Ludlam and Andrew J. Leigh Brown (1991), AIDS, pp. 675-681). It is also important to follow up the progress of HIV decrease in patients undergoing AIDS therapy.

Currently, an indirect detection method based on HIV antigen-antibody reaction has been established and become a major diagnostic means in laboratories. However, detection based on antigen-antibody reaction has been associated with the problem of a window period especially in screening. This is a time-lag between antigen infection and antigen or antibody production. For early clinical diagnosis of HIV infection, it is important to accurately, directly and rapidly detect the presence of HIV RNA in blood as an alternative to the indirect method based on antigen-antibody reaction. It is highly desirable to develop a new detection method that can be performed on smaller amounts of samples with higher sensitivity and at reduced cost and with a shortened window period. It is necessary to diagnose HIV infection from very small amounts of samples especially in infection in children or vertical infection.

Direct and rapid assay of HIV itself has become possible with the new introduction of molecular biological techniques such as polymerase chain reaction (PCR) or screening, for example, into clinical applications (Gerald Schochetman and John J. Sninsky (1991), "Direct Detection of Human Immunodeficiency Virus Infection Using the Polymerase Chain Reaction" Springer-Verlag pp. 90-110; Janet S. Bootman, Pete A. Kitchin (1994), J. Virological Methods, pp. 1-8; Anne-Mieke Vandamme, Sonia Van Dooren, Wessel Kok, Patrick Goubau, Katrien Fransen, Tim Kievits, Jean-Claude Schmit, Erik De Clercq, Jan Desmyter (1995), J. Virological Methods pp. 121-132; E. Lyamuya, U. Bredberg-Raden, J. Albert, O. Grankvist, V. Msangi, C. Kagoma, F. Mhalu, and G. Biberfield (1997), J. Clinical Microbiology, pp. 278-280). Such nucleic acid detection systems combining nucleic acid isolation with nucleic acid amplification reactions such as PCR consist of three distinct processes: i.e., nucleic acid isolation, amplification and detection. Nucleic acid isolation is an important process for subsequent amplification and detection, and if a nucleic acid isolation technique can be improved, the efficiency of amplification and detection will increase.

Currently, several types of total RNA isolation methods are known and a plurality of kits are commercially available (John M. Chirgwin, Alan E. Przybyla, Raymond J. MacDonald and William J. Rutter (1979), Biochemistry pp. 5294-5299; Osamu Yamada, Toshiya Matsumoto, Masahiro Nakashima, Shinobu Hagari, Toshio Kamahora, Hiroshi Ueyama, Yuichiro Kishi, Hidetoshi Uemura and Takashi Kurimura (1990), J. Virological Methods pp. 203-210).

However, these methods have disadvantages including the use of organic solvents such as phenol or chloroform to remove proteins and the necessity of changing tubes many times during the isolation process. Further, they are not ideal for isolation from very small amounts of samples, especially blood products or blood samples from children. They also have the disadvantage that costs add up when a number of samples are assayed in screening or the like. Thus, it has become increasingly important to develop a rapid and inexpensive HIV RNA detection system.

In recent years, several types of methods for isolating a nucleic acid, especially RNA have come to be known such as the guanidinium cyanate-phenol-chloroform (AGPC) method (Piotr Chomczynski, Nicolette Sacchi (1987), Analytical Biochemistry pp. 156-159) and methods using CsCl₂ or a resin (Maniatis T. et al.: Molecular Cloning: A laboratory manual, 2nd ed., Cold Spring Harbor 1989). Japanese Patent Public Disclosure No. 236499/1995 incorporated herein as reference discloses an improved AGPC method for isolating a viral nucleic acid, in which the isolation process can be completed in a single tube without using an organic solvent.

### SUMMARY OF THE INVENTION

An object of the present invention is to provide a nucleic acid isolation kit comprising a reducing agent, a coprecipitant and a protein denaturant characterized in that it contains no protease.

The nucleic acid isolation kit of the present invention is preferably an RNA isolation kit.

Another object of the present invention is to provide a method for isolating a nucleic acid from a biological sample, comprising:
i) incubating the biological sample with a reducing agent, a coprecipitant and a protein denaturant to degrade and denature proteins and other contaminants in the biological sample without using a protease, and
ii) directly performing alcohol precipitation with a lower alcohol.

### BRIEF DESCRIPTION OF DRAWINGS

FIG. 1 is a set of electrophoretic pattern of reaction products after nucleic acid isolation at varying glycogen concentrations and amplification.
FIG. 2 is a flowchart of a preferred embodiment of a nucleic acid isolation method of the present invention.
FIG. 3 shows the sequences of nucleic acids isolated by a method of the present invention.

### DETAILED DESCRIPTION OF THE INVENTION

Methods for nucleic acid detection consist of three main processes, i.e., nucleic acid isolation, amplification and detection. Improvements in the nucleic acid isolation process are also important for the subsequent amplification and detection processes.

Before the present invention was made, the isolation technique used in many nucleic acid detection methods was the AGPC method described above. The present inventors found a novel method for isolating a nucleic acid from a sample by using an improved AGPC method in combination with a coprecipitant, to accomplish the present invention. The details are described below.

### Nucleic acid isolation kits

Specifically, the present invention provides a nucleic isolation kit comprising a reducing agent, a coprecipitant and a protein denaturant characterized in that it contains no protease.

A key feature of the present invention is that no protease is used. In conventional methods, non-specific proteases such as proteinase K, pronase and subtilisin are first used to remove proteins from biological samples. In the present invention, nucleic acids can be efficiently isolated only with a coprecipitant and a protein denaturant without using such a protease.

### i) Reducing agents

In the present invention, reducing agents include, but are not limited to, 2-mercaptoethanol, dithiothreitol, etc. These reducing agents are used for protection of sulfhydryl groups in proteins or for reductive cleavage of disulfide bonds.

Reducing agents can be preferably used at a concentration of, but not specifically limited to, about 1%. Reducing agents may be added to nucleic acids as premixes in protein denaturants.

### ii) Coprecipitants

In the present invention, coprecipitants are not specifically limited to, and any coprecipitants that can function as carriers for coprecipitating nucleic acids are suitable. Preferred examples of coprecipitants are macromolecular polysaccharides such as glycogen and dextran.

Glycogen is a polymer of D-glucose having a molecular weight of one million to several millions and is known as one of the most potent carriers for nucleic acid precipitation (Steven Tracy (1981), Biochemistry pp. 251-268), and several types of commercially available glycogen can be used for in vitro applications. In the present invention, glycogen from slipper limpet is preferred from the viewpoints of cost and detection sensitivity.

The concentration of coprecipitants is not specifically limited, but as an example, glycogen from slipper limpet is used at 0.1 mg/ml to 2.0 mg/ml, preferably 0.2 mg/ml to 2.0 mg/ml, most preferably about 0.2 mg/ml (Example 2).

### iii) Protein denaturants

Suitable protein denaturants are those known to be able to solubilize proteins. They include, but not specifically limited to, guanidine thiocyanate and urea. Guanidine thiocyanate is especially preferred. A preferred embodiment of the present invention uses glycogen as a coprecipitant and guanidine thiocyanate as a protein denaturant, and is called the glycogen-guanidinium (GG) method.

Protein denaturants can be used at a final concentration of 3 M to 7 M, preferably 4 M to 6 M, most preferably approximately 4.5 M. Preferably, protein denaturants at the saturation concentration can solubilize proteins and efficiently reduce RNases that may be included in biological samples particularly in the case of isolation of RNA.

Preferably, protein denaturants are added to reactants after biological samples are mixed with coprecipitants. As described above, they may be added as premixes with reducing agents to reaction solutions.

Further, without ant limitation, protein denaturants can be optionally added to an alcohol during alcohol precipitation at the final stage of the isolation process to more clearly remove contaminant proteins especially in the case of samples containing a lot of contaminants such as blood products. Protein denaturants are added to an alcohol during the alcohol precipitation step preferably at 0.5 M to 2.0 M, most preferably about 0.9 M (Example 3).

In the present invention, there is no special need to externally add a salt during the nucleic acid isolation process, and nucleic acid isolation kits specially require no salt. Moreover, the pH need not be specially externally adjusted.

### Nucleic acid isolation methods

The present invention also provides methods for isolating a nucleic acid from a biological sample.

Methods of the present invention comprises:
i) incubating the biological sample with a reducing agent, a coprecipitant and a protein denaturant to degrade and denature proteins and other contaminants in the biological sample without using a protease, and
ii) directly performing alcohol precipitation with a lower alcohol.

In the present invention, nucleic acid isolation can be normally made from biological samples in a volume of about 50 µl, preferably 30 µl to 100 µl. This means that significantly smaller amounts of biological samples are required than conventional methods.

According to a preferred but non-limitative embodiment, a biological sample is mixed with a coprecipitant and then a reducing agent and a protein denaturant are added in step i).

In step i), incubation takes place at 55°C - 65°C, preferably about 60°C for 5 minutes to 15 minutes, preferably about 10 minutes. Most preferably, incubation takes place at about 60°C for about 10 minutes. Incubation can be performed in an apparatus such as, but not limited to, an incubator, PCR apparatus (eg, Thermal Cycler® from Perkin-Elmer), etc.

In step ii), the reaction mixture obtained in step i) is then combined with a lower alcohol to precipitate a nucleic acid. Alcohol precipitation can be performed by known techniques. Suitable alcohols include isopropanol and ethanol or the like. Preferably, isopropanol is added at a final concentration of 40% or more or ethanol is added at a final concentration of 70% or more. Then, the mixture may be cooled at -70°C to 4°C after addition of a lower alcohol to promote efficacy of salting out.

A protein denaturant may be added during alcohol precipitation as described above. This can remove contaminants precipitating with a target nucleic acid.

Alcohol precipitation is followed by centrifugation at 14,000 x g to 19,000 x g for 5 minutes to 15 minutes. Centrifugation may be performed at room temperature or under cooling at about 4°C. After centrifugation, the supernatant is decanted or sucked to recover the precipitated nucleic acid.

Finally, the obtained nucleic acid can be washed with 70% ethanol, for example, and redissolved in a suitable solution into a ready-to-use state.

All the steps of the isolation process described above can be performed in a single tube. This can prevent contamination which may otherwise be caused by a change of tubes during the isolation process. This is especially important particularly when the nucleic acid is amplified by PCR or the like. Moreover, the isolation process of the present invention can be performed in a 0.5 ml tube in contrast to conventional methods that required a tube of at least 1.5 ml or more in size. Thus, the necessary amount of each reagent can be reduced and also the nucleic acid redissolved in a 0.5 ml tube can be directly used for amplification reaction.

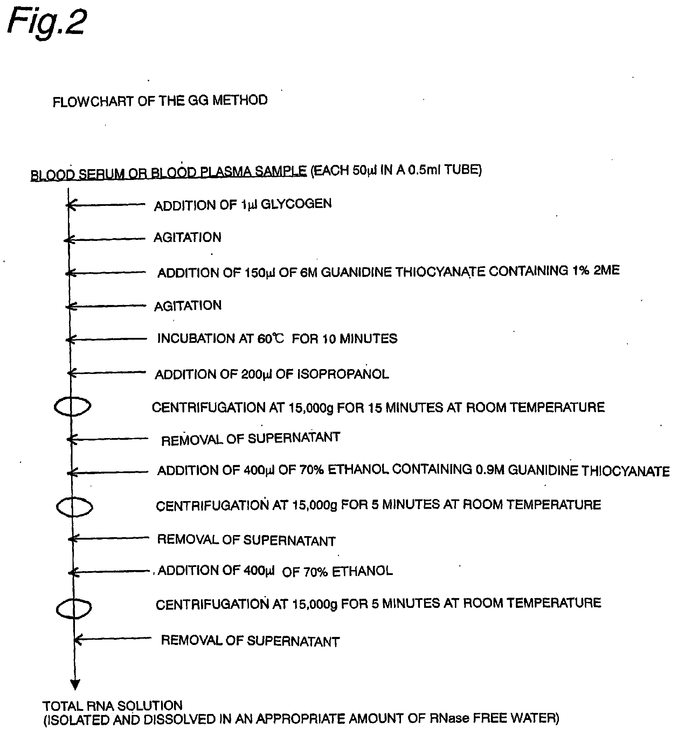

A preferred but non-limitative embodiment of the present invention is shown in Example 1 and Fig. 2.

Nucleic acid isolation methods of the present invention described above can be applied to isolate both DNA and RNA. Preferably, they can be used for RNA isolation. In addition to RNA of the AIDS virus HIV described in the examples below, they can be clinically applied to type C hepatitis virus (HCV), influenza virus, type A hepatitis virus and the like. In addition to blood samples such as serum and plasma, they are also useful for soluble liquid samples such as cerebrospinal fluid, saliva, semen and urine. They can also be used for powdered blood products derived from plasma or serum so that they have wide applications. Particularly, they are effective for small amounts of samples such as samples from children or powdered blood products.

Nucleic acids isolated by methods of the present invention can be subsequently amplified and detected, if necessary. That is, methods of the present invention can be combined with amplification techniques such as PCR to detect even very small amounts of nucleic acids at low concentrations that could not be detected by conventional methods.

### Amplification

### (1) Reverse transcription reaction

When the target nucleic acid is an RNA such as viral RNA, the RNA can be reversely transcribed with a reverse transcriptase capable of converting the RNA into cDNA directly in the tube used for isolation. That is, the RNA isolated by methods of the present invention is highly pure and contains no substances inhibiting reverse transcription reaction. Suitable reverse transcriptases include, for example, a reverse transcriptase derived from avian myeloblastosis virus used in Example 1 described below.

### (2) Amplification of nucleic acids

Nucleic acids isolated by methods of the present invention can be amplified by known nucleic acid amplification reactions such as polymerase chain reaction (PCR) using primers specific to the nucleic acids directly in the case of DNA or after reverse transcription into cDNA in the case of RNA. Nucleic acids isolated by methods of the present invention are highly pure and they contain no substances inhibiting amplification reaction even though ordinary PCR techniques require strict conditions.

Amplification reaction of nucleic acids may be nested PCR involving first PCR with an outer primer pair of the target nucleic acid followed by second PCR with an inner primer pair as described in Example 1 below, for example. The extent of amplification may vary with the affinity of the primer for the target nucleic acid. In HIV-1 described in the examples below, for example, gag primers and pol primers gave somewhat different extents of amplification and pol primers showed a higher detection sensitivity than gag primers.

### Detection

Amplified nucleic acids can be detected and identified by standard techniques such as electrophoresis and sequencing. For example, they can be detected by ethidium bromide staining after electrophoresis on a polyacrylamide gel or an agarose gel. The sequence can also be identified by known methods using commercially available nucleic acid sequencers.

As described above, methods of the present invention achieve high cost efficiency, high sensitivity, safety and time reduction in nucleic acid isolation. They can be combined with amplification techniques such as PCR to provide accurate, direct and rapid diagnosis for virus infection, for example. The GG method of the present invention can be applied not only to the field of research but also to the clinical field.

The following examples further illustrate the present invention but are not intended to limit the technical scope of the invention. Those skilled in the art can readily add modifications/changes to the present invention on the basis of the description of the specification, and those modifications/changes are included in the technical scope of the present invention.

### EXAMPLES

In the examples of the present invention, the following materials were used unless otherwise specified.

### Materials

### (1) Samples

In both AMPLICOR HIV-1 MONITOR® Test Kit (Roche) and NASBA Amplification System (Organon Teknika, Boxtel, Netherlands), 26 samples containing high copy numbers of HIV-1 RNA (30,000 copies or more / ml) and 47 samples containing low copy numbers below the detection limit (1,000 copies or less / ml) were used.

### (2) Glycogen

Oyster glycogen (Sigma), slipper limpet glycogen (Sigma), rabbit glycogen (Sigma) or bovine glycogen (Sigma) was used.

### (3) RNA isolation methods used as controls

The following commercially available 7 kits/methods were used as controls of the present invention in the examples.
TRIZOL LS®
ISOGEN LS® (Nippon Gene)
RNA Isolation Technique (Stratagene)
SepaGene-RV® (Sanko Junyaku)
NASBA RNA Isolation Kit (Organon Teknika)
Smitest® (SUMITOMO METAL INDUSTRIES., LTD)
Cartrimox (Iowa Biotechnology).

### Example 1: RNA isolation by the GG method and amplification and detection of the isolated RNA

HIV-1 RNA was isolated, amplified and detected from HIV-1-containing samples by the following procedures.

### a. RNA isolation

RNA was isolated from samples following the scheme shown in Fig. 2 as an example of the GG method of the present invention.

In a 0.5 ml tube, 1 µl of glycogen (10 mg/ml) was added to 50 µl of each sample and the tube was agitated. The tube was incubated at 60°C for 10 minutes with 150 µl of 6 M guanidine thiocyanate containing 1% 2-mercaptoethanol (2ME). Then, 200 µl of isopropanol was added. The tube was centrifuged at 15,000 g for 15 minutes at room temperature, and then the supernatant was removed. 400 µl of 70% ethanol containing 0.9 M guanidine thiocyanate was added. The tube was centrifuged at 15,000 g for 5 minutes at room temperature, and then the supernatant was removed. 400 µl of 70% ethanol was added. The tube was centrifuged at 15,000 g for 5 minutes at room temperature, and then the supernatant was removed. The residue was dissolved in 10 µl of RNase-free sterile water.

### b. Amplification

### 1) Primers

For amplification of HIV-1 RNA, gag and/or pol primers (SEQ ID NOS: 1-8) shown in Table 1 below were used (derived from a human retrovirus of AIDS obtained from Los Alamos National Laboratory).

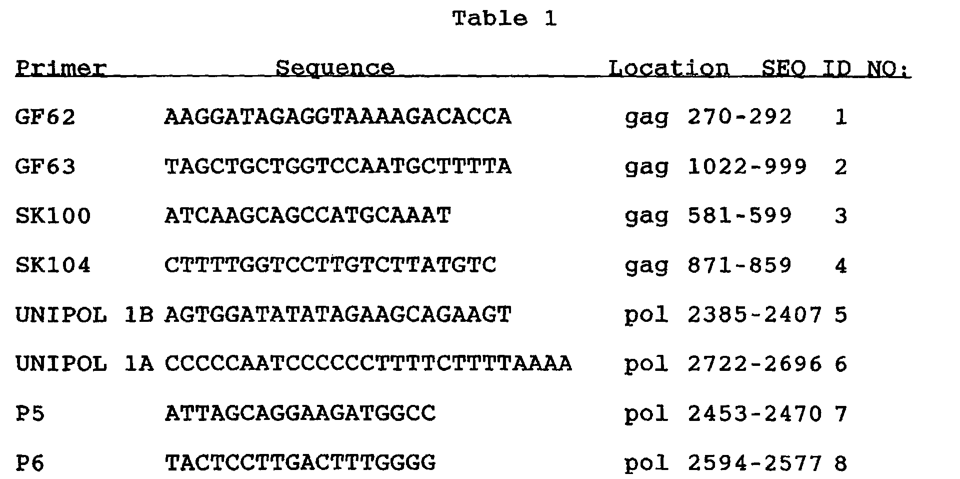

The primer pair GF62 and GF63 and the primer pair UNIPOL 1B and UNIPOL 1A are outer primer pairs of gag and pol, respectively. On the other hand, the primer pair SK100 and SK104 and the primer pair P5 and P6 are inner primer pairs of gag and pol, respectively.

### 2) PCR

The RNA isolated as above was used for nucleic acid amplification by a conventional PCR method.
i) Initially, 2 drops of mineral oil was added to a 0.5 ml tube containing the above isolated RNA dissolved in 10 µl of sterile water and the tube was momentary spun. Then, the tube was incubated at 80°C for 10 minutes in a Thermal Cycler® (Perkin-Elmer) to destroy any RNA complexes or secondary structures that may disturb priming in PCR. After 10 minutes, the tube was immediately transferred into ice to stop the reaction and momentary spun.
ii) Then, 15 µl of a mixture for RT having the composition shown in Table 2 below was added and the tube was vortexed and momentary spun.

**TABLE 2**

| Composition of mixture for RT | |
|---|---|
| H₂O | 7.13 |
| x 10 PCR buffer | 2.5 |
| 10 mM MgCl₂ | 2.0 |
| 10 mM DTT | 0.5 |
| 2.5 mM dNTP | 1.5 |
| 1 µl/ml Primer 1 (outer) | 0.25 |
| 1 µl/ml Primer 2 (outer) | 0.25 |
| 10 U/µl RNase inhibitor | 0.77 |
| 2.5 U/µl Reverse transferase (AMVRT) | 0.1 |
| Total | 15.00 µl |

The tube was incubated in a warm bath (42°C) for 60 minutes to synthesize cDNA from mRNA. Then, the tube was momentary spun.
iii) Then, the tube was incubated at 99°C for 6 minutes in a Thermal Cycler to denature double stranded mRNA-cDNA into single strands. After 6 minutes, the tube was immediately transferred into ice to stop the reaction and momentary spun.
iv) Then, 75 µl of a mixture for first PCR having the composition shown in Table 3 below was added and the tube was vortexed and momentary spun.

**TABLE 3**

| Composition of mixture for PCR | |
|---|---|
| H₂O | 58 |
| x 10 PCR buffer | 7.5 |
| 10 mM MgCl₂ | 3.0 |
| 2.5 mM dNTP | 4.5 |
| 1 µl/ml Primer 1 (outer) | 0.75 |
| 1 µl/ml Primer 2 (outer) | 0.75 |
| Taq polymerase | 0.5 |
| Total | 75.00 µl |

First PCR was performed in a Thermal Cycler. Specifically, 30 cycles of 94°C for 30 seconds, 55°C for 30 seconds and 72°C for 1 minute were performed. Then, the tube was incubated at 72°C for 10 minutes and then, the reaction was stopped at 4°C.
v) Then, 10 µl of the first PCR product obtained in iv) and mineral oil were added to a fresh 0.5 ml tube, and the tube was momentary spun. Then, the tube was incubated at 99°C for 6 minutes in a Thermal Cycler to denature double-stranded DNA into single strands. After 6 minutes, the tube was immediately transferred into ice to stop the reaction and momentary spun.
vi) Then, 90 µl of a mixture for second PCR having the composition shown in Table 4 below was added and the tube was vortexed and momentary spun.

**Table 4.**

| Composition of mixture for second PCR | |
|---|---|
| H₂O | 66.5 |
| x 10 PCR buffer | 10 |
| 10 mM MgCl₂ | 5 |
| 2.5 mM dNTP | 6 |
| 1 µl/ml Primer 3 (inner) | 1 |
| 1 µl/ml Primer 4 (inner) | 1 |
| Taq polymerase | 0.5 |
| Total | 90.0 µl |

Second PCR was performed in a Thermal Cycler. Specifically, 30 or 40 cycles of 94°C for 30 seconds, 55°C for 30 seconds and 72°C for 1 minute were performed. Then, the tube was incubated at 72°C for 10 minutes and then, the reaction was stopped at 4°C.

### c. Detection

### Electrophoresis and staining

Following amplification, PCR products were electrophoresed. Specifically, a dye was added to the second PCR product and an aliquot of 10 µl was loaded on a 5% acrylamide gel or a 2% agarose gel and electrophoresed. HaeIII digest of ΦX174 (Roche) was used as a molecular weight marker. Culture supernatant of an HIV-1 infected cell line MOLT-4/HTLV III was used as a positive control. After completion of migration, bands were visualized by ethidium bromide staining.

### Example 2: Optimal concentration of glycogen used for RNA isolation

The concentration of glycogen used as a carrier was diversified to determine the optimal concentration from the viewpoints of isolation efficiency, time reduction and cost efficiency.

Specifically, samples containing low copy numbers of HIV-1 defined above were used to isolate RNA by the GG method of the present invention described above except that 1 µl each of glycogen at a concentration of 0, 5, 10, 50 or 100 mg/ml was used and the concentration was changed to a final value of 0, 0.1, 0.2, 1 or 2 mg/ml. Isolated RNA was amplified over 30 or 40 cycles of PCR. The primers used for amplification were a gag primer pair of SK 100 and SK 104 and a pol primer pair of P5 and P6. Theoretically, it is expected that 291 bp and 142 bp of PCR products are obtained with the gag and pol primer pairs, respectively.

One µl of a dye was added to 9 µl of the second PCR product and the mixed product was loaded on a 5% polyacrylamide or 2.0% agarose gel and electrophoresed. Bands were detected by ethidium bromide staining.

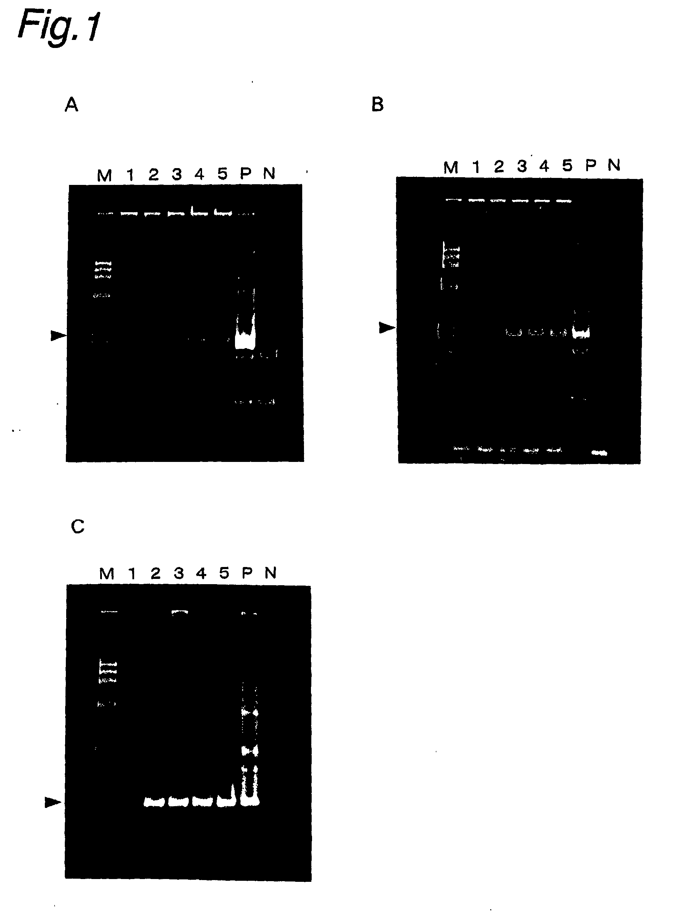

The results are shown in Fig. 1. In Fig. 1, panels A, B and C show the results after 30 cycles with the gag primer pair, 40 cycles with the gag primer pair and 30 cycles with the pol primer pair, respectively. Lanes 1-5 in each panel represent bands at glycogen concentrations of 0 mg/ml, 0.1 mg/ml, 0.2 mg/ml, 1 mg/ml and 2 mg/ml, respectively. Lane 6: positive control (P); lane 7: negative control (N); M: molecular weight marker (Roche).

As shown in Fig. 1, bands were detected at glycogen concentrations of 0.2 mg/ml to 2 mg/ml (lanes 3-5) in each case. Therefore, glycogen can function as a carrier at a concentration of about 0.2 mg/ml or more.

### Example 3: Optimal concentration of guanidine thiocyanate used for RNA isolation

The concentration of guanidine thiocyanate added to 70% ethanol during the final ethanol washing step was changed to determine the optimal concentration from the viewpoint of isolation efficiency.

Specifically, samples containing low copy numbers of HIV-1 defined above were used to isolate RNA by the GG method of the present invention described above except that the concentration of guanidine thiocyanate added to 70% ethanol during the final ethanol washing step was changed to 0.0 M, 0.6 M, 0.7 M, 0.8 M, 0.9 M and 1.0 M.

As a result, small amounts of contaminants remained as masses in the final RNA at a guanidine thiocyanate concentration of 0.8 M or less but disappeared at 0.9 M or more.

### Example 4: Comparison of RNA isolation efficiency

The efficiency of the GG method of the present invention was compared with those of commercially available known RNA or DNA isolation kits on HIV samples.

Specifically, seven widely used commercial RNA or DNA isolation kits were adopted as controls to perform nucleic acid isolation according to the ordinary protocol of each kit. For each kit, 26 high copy-number samples and 47 low copy-number samples described above were used. Nucleic acids were amplified by PCR with the gag primer pair and/or pol primer pair described above and detected as described above.

The results are shown in Table 5 below.

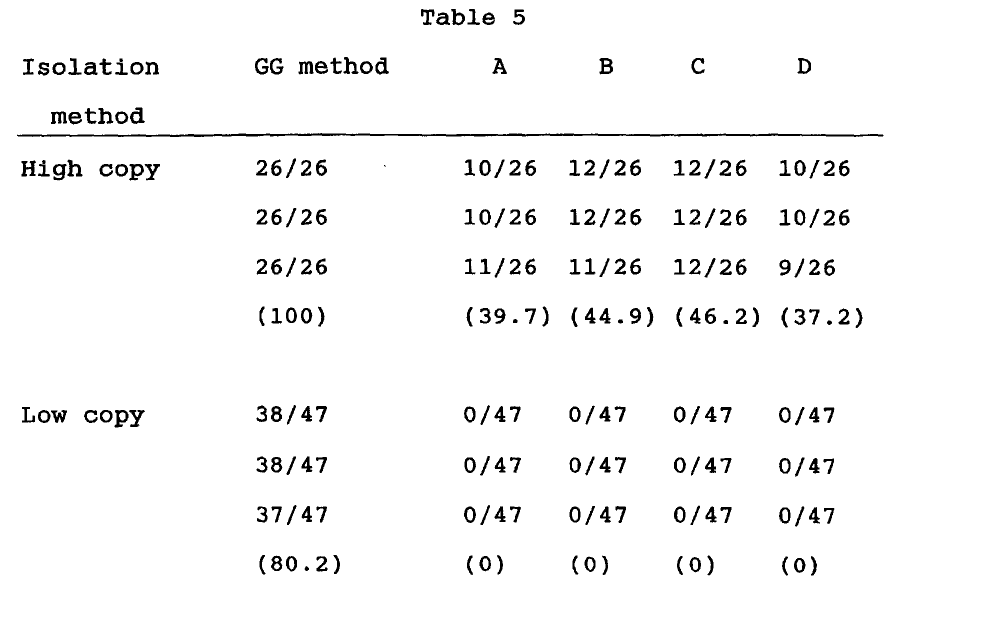

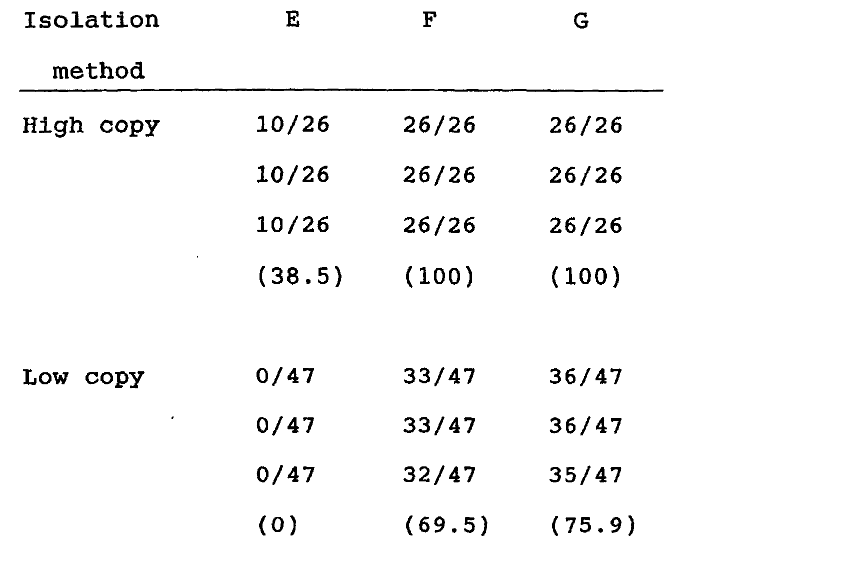

In high copy-number samples, detection was about 35% to about 50% with control kits A to E and 100% with F or G kit. In contrast, the method of the present invention achieved detection of 100%. In low copy-number samples, detection was 0% with control kits A to E and about 70% with F or G kit in contrast to the method of the present invention that achieved detection of 80.2% even in low copy-number samples.

This demonstrates that the present invention shows significantly excellent isolation efficiency as compared with many existing RNA isolation methods.

### Example 5: Identification of the sequences of amplified nucleic acids

Amplified samples were sequenced.

Specifically, an automated sequencer ABI-PRISM® 310 (Perkin-Elmer) was used for direct sequencing as recommended by the manufacturer. The target region in this Example is the gag or pol region of HIV-1. The reference sequence was retrieved from the annual report of the Los Alamos National Laboratory. Phylogenetic trees were used for data analysis.

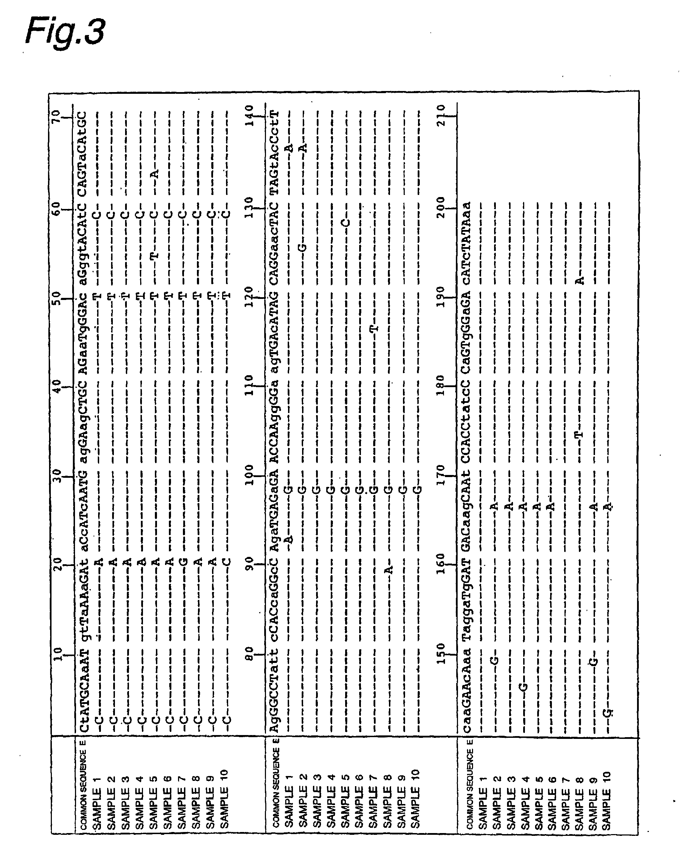

The results on PCR products using the gag primer pair are shown in Fig. 3 and SEQ ID NOS: 10-19. The nucleotide sequences of nucleic acids can be determined in all the samples subjected to RNA isolation by the GG method of the present invention. Sequencing of 10 samples revealed a common sequence of subtype E (Fig. 3). Sequence data show that all the 10 samples tested have a 90% or more homology with the common sequence (SEQ ID NO: 9) in the gag region of HIV-1 (291 bp). In Fig. 3, bases identical to those of the common sequence are shown by "-".

This verifies that each sample subjected to nucleic acid isolation contains HIV-1 RNA and that the presence of HIV-1 was detected by isolation and amplification of the RNA.

### EFFECT OF THE INVENTION

We compared known isolation methods using commercial kits with a method of the present invention. The results showed that the method of the present invention was the most excellent isolation method from the viewpoints of detection sensitivity, economy and time reduction.

Conventional methods using CsCl₂ or a resin were suitable in terms of time reduction, but inferior to the method of the present invention in detection sensitivity. The method of the present invention is safer than the AGPC method because it uses neither phenol nor chloroform. Moreover, the necessary period is within 60 minutes, which is shorter than required by conventional methods because any organic solvents such as phenol and chloroform are not used. The method of the present invention can be performed more inexpensively on small amounts (about 50 µl) of samples. All the steps of the method of the present invention can be performed in a 0.5 ml tube with no need to change the tube. Thus, processes from nucleic acid isolation to amplification can be completed in a single step, whereby the risk of contamination of reaction products is decreased.

Without being bound to any theory, the excellent isolation efficiency of the method of the present invention is partially attributed to the use of glycogen as a carrier of nucleic acids. The present invention is particularly useful for screening small amounts of samples such as blood products or blood samples from children, for example, and for following up patients under AIDS therapy, and it is useful for not only the field of research but also clinical applications.

## Claims

1. A nucleic acid isolation kit comprising a reducing agent, a coprecipitant and a protein denaturant **characterized in that** it contains no protease.

2. The nucleic acid isolation kit of Claim 1, which is salt-free.

3. The nucleic acid isolation kit of Claim 1 or 2
wherein the nucleic acid is RNA.

4. The nucleic acid isolation kit of any one of Claims 1 to 3 wherein the reducing agent is 2-mercaptoethanol or dithiothreitol.

5. The nucleic acid isolation kit of any one of Claims 1 to 4 wherein the coprecipitant is glycogen or dextran.

6. The nucleic acid isolation kit of any one of Claims 1 to 5 wherein the protein denaturant is guanidine thiocyanate.

7. A method for isolating a nucleic acid from a biological sample, comprising:
i) incubating the biological sample with a reducing agent, a coprecipitant and a protein denaturant to degrade and denature proteins and other contaminants in the biological sample without using a protease, and
ii) directly performing alcohol precipitation with a lower alcohol.

8. The method of Claim 7 wherein incubation takes place at 55°C to 65°C for 5 minutes to 15 minutes in step i).

9. The method of Claim 8 wherein incubation takes place at about 60°C for about 10 minutes in step i).

10. The method of any one of Claims 7 to 9 comprising adding a protein denaturant during alcohol precipitation.

11. The method of any one of Claims 7 to 10 wherein the biological component is a body fluid or a blood product.

12. The method of any one of Claims 7 to 10 wherein the biological sample has a volume of 30 µl to 100 µl.

13. The method of any one of Claims 7 to 11 comprising no step of adding a salt.

14. The method of any one of Claims 7 to 12 wherein said steps are performed in a single 0.5 ml tube.


SEQUENCE LISTING

<110> Oriental Yeast Co., Ltd.
NAITOU Toshikuni
National Institute of Infectious Diseases
TAKEDA Yoshifumi
<120> Kits for extracting nucleic acid and method of extracting
nucleic acid by using the kits
<130> YCT513
<160> 19

<210> 1
<211> 23
<212> DNA
<213> Artificial Sequence
<400> 1
aaggatagag gtaaaagaca cca 23

<210> 2
<211> 23
<212> DNA
<213> Artificial Sequence
<400> 2
tagctgctgg tccaatgctt tta 23

<210> 3
<211> 19
<212> DNA
<213> Artificial Sequence

<400> 3
atcaagcagc catgcaaat 19

<210> 4
<211> 22
<212> DNA
<213> Artificial Sequence
<400> 4
cttttggtcc ttgtcttatg tc 22

<210> 5
<211> 23
<212> DNA
<213> Artificial Sequence
<400> 5
agtggatata tagaagcaga agt 23

<210> 6
<211> 27
<212> DNA
<213> Artificial Sequence
<400> 6
   cccccaatcc ccccttttct tttaaaa 27

<210> 7
<211> 18
<212> DNA
<213> Artificial Sequence
<400> 7

attagcagga agatggcc    18

<210>  8
<211>  18
<212>  DNA
<213>  Artificial Sequence
<400>  8
tactccttga ctttgggg    18

<210>  9
<211>  220
<212>  DNA
<213>  HIV-1
<400>  9
ctatgcaaat gttaaaagat accatcaatg aggaagctgc agaatgggac    50
agggtacatc cagtacatgc agggcctatt ccaccaggcc agatgagaga    100
accaagggga agtgacatag caggaactac tagtaccctt caagaacaaa    150
taggatggat gacaagcaat ccacctatcc cagtgggaga catctataaa    200
agatggataa tcctgggatt                                    220

<210>  10
<211>  220
<212>  DNA
<213>  HIV-1
<400>  10
ccatgcaaat gttaaaagaa accatcaatg aggaagctgc agaatgggat    50
agggtacacc cagtacatgc agggcctatt ccaccaggcc aaatgaggga    100
accaagggga agtgacatag caggaactac tagtaacctt caagaacaaa    150
taggatggat gacaagcaat ccacctatcc cagtgggaga catctataaa    200

aggtggataa tcctgggatt 220

<210> 11
<211> 220
<212> DNA
<213> HIV-1
<400> 11
ccatgcaaat gttaaaagaa accatcaatg aggaagctgc agaatgggat 50
agggtacacc cagtacatgc agggcctatt ccaccaggcc agatgaggga 100
accaagggga agtgacatag cagggactac tagtaacctt caagaacaga 150
taggatggat gacaaacaat ccacctatcc cagtgggaga catctataaa 200
aggtggataa tcctgggatt 220

<210> 12
<211> 220
<212> DNA
<213> HIV-1
<400> 12
ccatgcaaat gttaaaagaa accatcaatg aggaagctgc agaatgggat 50
agggtacacc cagtacatgc agggcctatt ccaccaggcc agatgaggga 100
accaagggga agtgacatag caggaactac tagtaccctt caagaacaaa 150
taggatggat gacaaacaat ccacctatcc cagtgggaga catctataaa 200
agatggataa tcctgggatt 220

<210> 13
<211> 220
<212> DNA
<213> HIV-1
<400> 13

ccatgcaaat gttaaaagaa accatcaatg aggaagctgc agaatgggat 50
agggtacacc cagtacatgc agggcctatt ccaccaggcc agatgaggga 100
accaagggga agtgacatag caggaactac tagtaccctt caagagcaaa 150
taggatggat gacaaacaat ccacctatcc cagtgggaga catctataaa 200
aggtggataa tcctgggatt 220

<210> 14
<211> 220
<212> DNA
<213> HIV-1
<400> 14
ccatgcaaat gttaaaagaa accatcaatg aggaagctgc agaatgggat 50
aggttacacc caatacatgc agggcctatt ccaccaggcc agatgaggga 100
accaagggga agtgacatag caggaaccac tagtaccctt caagaacaaa 150
taggatggat gacaaacaat ccacctatcc cagtgggaga catctataaa 200
aggtggataa tcctgggatt 220

<210> 15
<211> 220
<212> DNA
<213> HIV-1
<400> 15
ccatgcaaat gttaaaagaa accatcaatg aggaagctgc agaatgggat 50
agggtacacc cagtacatgc agggcctatt ccaccaggcc agatgaggga 100
accaagggga agtgacatag caggaactac tagtaccctt caagaacaaa 150
taggatggat gacaaacaat ccacctatcc cagtgggaga catctataaa 200
aggtggataa tcctgggatt 220

<210> 16

<211> 220
<212> DNA
<213> HIV-1
<400> 16
ccatgcaaat gttaaaagag accatcaatg aggaagctgc agaatgggat 50
agggtacacc cagtacatgc agggcctatt ccaccaggcc agatgaggga 100
accaagggga agtgatatag caggaactac tagtaccctt caagaacaaa 150
taggatggat gacaagcaat ccacctatcc cagtgggaga catctataaa 200
aggtggataa tcctgggatt 220

<210> 17
<211> 220
<212> DNA
<213> HIV-1
<400> 17
ccatgcaaat gttaaaagaa accatcaatg aggaagctgc agaatgggat 50
agggtacacc cagtacatgc agggcctatt ccaccaggac agatgaggga 100
accaagggga agtgacatag caggaactac tagtaccctt caagaacaaa 150
taggatggat gacaagcaat ccatctatcc cagtgggaga aatctataaa 200
aggtggataa tcctgggatt 220

<210> 18
<211> 220
<212> DNA
<213> HIV-1
<400> 18
ccatgcaaat gttaaaagaa accatcaatg aggaagctgc agaatgggat 50
agggtacacc cagtacatgc agggcctatt ccaccaggcc agatgaggga 100
accaagggga agtgacatag caggaactac tagtaccctt caagaacaga 150

taggatggat gacaaacaat ccacctatcc cagtgggaga catctataaa 200
aggtggataa tcctgggatt 220

<210> 19
<211> 220
<212> DNA
<213> HIV-1
<400> 19
ccatgcaaat gttaaaagac accatcaatg aggaagctgc agaatgggat 50
agggtacacc cagtacatgc agggcctatt ccaccaggcc agatgaggga 100
accaagggga agtgacatag caggaactac tagtaccctt caggaacaaa 150
taggatggat gacaaacaat ccacctatcc cagtgggaga catctataaa 200
aggtggataa tcctgggatt 220